# EUROPEAN PATENT SPECIFICATION

(11) **EP 0 595 805 B1**
(45) Date of publication and mention of the grant of the patent: **17.09.1997**
(21) Application number: 91912431.3
(22) Date of filing: 19.06.1991
(51) Int. Cl.: A61L 33/00

(54) **METHOD OF MODIFYING THE PROPERTIES OF A SUBSTRATE SURFACE BY COVALENT BONDING OF A COMPOUND TO THE SURFACE, AND MEMBRANE MODIFIED ACCORDING TO THIS METHOD**
VERFAHREN ZUR MODIFIZIERUNG DER OBERFLÄCHE EINES SUBSTRATES DURCH KOVALENTE BINDUNG EINER VERBINDUNG AN DIESEM SUBSTRAT, UND DURCH DIESEM VERFAHREN MODIFIZIERTE MEMBRAN
PROCEDE PERMETTANT DE MODIFIER LES PROPRIETES DE LA SURFACE D'UN SUBSTRAT PAR LIAISON COVALENTE D'UN COMPOSE SUR LA SURFACE, ET MEMBRANE MODIFIEE SELON CE PROCEDE

(30) Priority: 19.06.1990 NL 9001387
(43) Date of publication of application: 11.05.1994
(73) Proprietor: HOLLAND BIOMATERIALS GROUP B.V., NL-7522 NB Enschede (NL)
(72) Inventor: ENGBERS, Gerardus, Henricus, Maria, NL-7577 AP Oldenzaal (NL); FEIJEN, Jan, NL-7552 GD Hengelo (NL)
(74) Representative: Smulders, Theodorus A.H.J., Ir.
(86) International application number: NL9100103
(87) International publication number: WO9119521

## Description

The invention relates to a method of modifying the properties of a surface of a substrate by covalent bonding of a physiologically active compound solved in a solvent-containing solution and containing one or more functional groups to the surface. Such methods are known from EP-A-46828 and EP-A-336964.

According to EP-A-46828 surfaces of substrates are made antithrombogenic by treating with heparin and a bridging substance bound covalently with heparin and the substrate. That is to say acrylic acid is esterified with the OH groups of heparin on the one side and polymerised with the substrate on the other side.

According to EP-A-336964 surfaces of substrates are made antithrombotic by reacting the amino group of heparin with a polyepoxide and the other epoxy part with the substrate.

The known methods have the disadvantage that addition reaction of carbon double bonds or α-OH resulting from the opening of 1,2 epoxy bond may occur. This means that an undesired cross-linking reaction with heparin or the substrate may take place. Furthermore there is formed a bridging group containing more than 1 carbon atom between the substrate and the compound immobilized thereon. Since the properties of the surface are determined by, among others, the chemical composition of the surface, said bridging group is of an undisired deciding importance as to the ultimate properties of the substrate surface whereas preferably these properties only depend on heparin and the substrate.

The method according to the invention provides a solution to this problem and is characterized in that the one or more functional groups of said compound are all or partly activated by a reagent in a solvent containing first solution, whereafter the activated compound is exposed to a functional substrate surface in a solvent containing second solution during which the compound becomes covalently bonded to said surface, said reagent being a carbonylating reagent having a structural formula R₁-(CO)-R₂, wherein R₁ and R₂ represent groups which can easily be separated. Examples of such a reagent are 1,1'-carbonyl diimidazole or 1,1-carbonyldi-1,2,4-triazole. The choice of the reagent is determined *inter alia* by the nature of the functional group(s) of the compound and of the substrate and by the choice of the solvents to be used.

Apart from the feature that the compound contains one or more functional groups, the compound can be any compound which, after immobilization, changes the properties of the substrate surface involved, when in contact with a particular environment. Examples are compounds which, after immobilization, increase the biocompatibility of the substrate surface, for instance heparin.

The method is particularly suitable when the compound is a polyelectrolyte or a polyelectrolyte-containing compound having more than one functional group, for instance heparin. With these compounds, during activation of the compound, no cross-linking of the compound occurs, which adds an extra advantage to the method.

The compound is activated in a solvent suitable for activation, which solvent is not reactive with respect to the bifunctional reagent and the activated compound. The compound is immobilized in a solvent suitable for the immobilization of a sufficient amount of the compound to be immobilized. Examples of suitable solvents for activation in the case where the bifunctional reagent is a carbonylating reagent of the general structural formula R₁-(CO)-R₂, for example, 1,1'-carbonyl diimidazole or 1,1-carbonyl-1,2,4-triazole, are dioxane, tetrahydrofuran, acetone, formamide, dimethyl formamide and dichloromethane. The choice of the solvent during activation of the compound is also based on the solubility of the compound. The choice of the solvent during immoblization is also determined by the interaction of the substrate surface with the solvent and by the solubility of the activated compound in the solvent. Mixtures of several solvents can also be used.

By way of example, the compound can be activated in a solution with a solvent of an organic nature, which solution, after the required reaction time, is added to the substrate surface which is in an aqueous environment.

As regards the compound, it may be necessary to modify it in such a way that its solubility in the selected solvent is increased. If the compound is a polyelectrolyte, this can be done by an appropriate choice of the counterion.

When a substrate surface to be modified does not have the required reactive groups it is possible to provide the surface in question with such groups beforehand.

In order to optimize the interaction of the compound after immobilization with components from the environment to which the substrate is exposed, it is possible to provide the surface beforehand with so-called "spacer" molecules, which increase the distance between the substrate surface and the compound. An example of a suitable spacer molecule is a functional poly(ethylene oxide)-containing molecule which, after bonding to the substrate surface, contains one or more fucntional groups, preferably a hydroxyl- and/or carboxyl- and/or amino group(s).

In addition to the advantage realized by the invention, namely obtaining a substrate surface with modified properties, a further advantage is that by first activating the compound there is no bifunctional reagent available during the immobilization step to react with the substrate surface. This makes the method advantageously suitable for modifying hydrophilic substrates, of which only the properties of the substrate surface change upon modification according to the method. Examples of such hydrophilic substrates are cellulose-based membranes and hydrogels.

The method also has the advantage that when the compound to be bonded is a polyelectrolyte-containing compound having more groups per molecule which are reactive with respect to the bifunctional reagent, for example heparin, cross-linking of the polyelectrolyte does not occur during the activation of said polyelectrolyte. The advantage is that the activity of the compound is retained after immobilization. Furthermore, the method is so simple that the surface modification with the activated compound can be carried out as a one-step procedure.

The invention also relates to a membrane having a surface modified in accordance with the method of the invention, preferably a cellulose membrane wherein the compound to be activated is heparin or a heparin-analogue.

In accordance with a further elaboration of the invention, hydroxyl- and/or carboxyl groups of the heparin are first activated with a bifunctional reagent, preferably a carbonylating reagent of the general structural formula R₁-(CO)-R₂, more preferably 1,1'-carbonyl diimidazole or 1,1-carbonyl-1,2,4-triazole. The activation takes place in a solvent which is not reactive with respect to the bifunctional reagent and the activated heparin, for example formamide. During this activation no cross-linking of the heparin occurs. Subsequently, the membrane is incubated in the solution of the activated heparin. After rinsing and drying the heparinized membrane is obtained.

In addition to the advantage accomplished by the invention, namely a strong improvement of the blood compatibility of the membrane, a further advantage is that by pre-activating the heparin with the reagent, during the immobilization step the reagent is not available for cross-linking the membrane, so that the permeability of the membrane involved is not affected by cross-linking of the membrane by the bifunctional reagent.

### EXAMPLE

Heparin sodium salt is converted into heparin-benzyl trimethyl ammonium salt via an ion-exchange procedure in order to increase the solubility of heparin in organic solvents. Subsequently, the heparin salt is dissolved in formamide (0.25 g.ml⁻¹) to which carbonyl diimidazole (CDl) is added until a heparin/CDl ratio (w/w) of 8.5 has been reached. After stirring for half an hour at room temperature, a cellulose membrane is added whereafter the reaction mixture is regularly homogenized for 48 hours. Thereafter, the membrane is rinsed with formamide, water and a NaCl solution and subsequently incubated in a glycerol solution and dried.

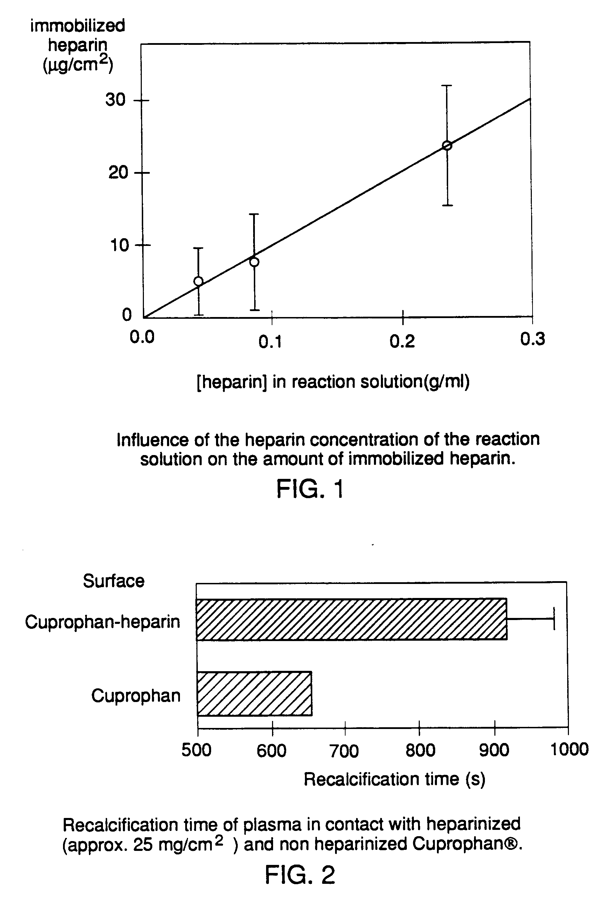

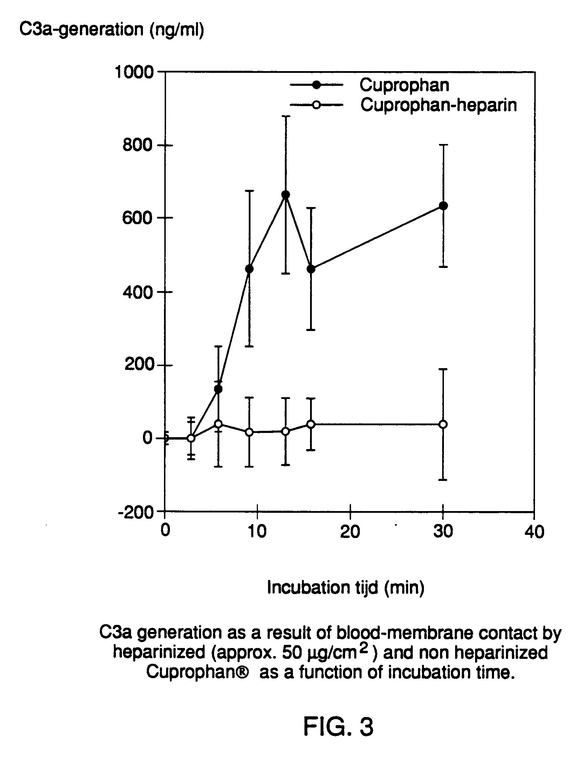

The heparinized membrane has a typical heparin surface concentration of about 50 µg.cm⁻². This amount can be varied controllably by adjusting the heparin concentration in the reaction mixture (see Fig. 1) The heparinized membrane has the same permeability to urea as the non-modified membrane. The blood compatibility of the membrane has been highly improved by the heparinization procedure. In comparison with citrate plasma which is incubated in non-modified membranes, citrate plasma which is incubated in the heparinized membranes shows a prolonged coagulation time (see Fig. 2). Further, heparinized membranes, unlike non-modified membranes, hardly, if at all, give rise to complement activation when they are contacted with blood (see Fig. 3).

## Claims

1. A method of modifying the properties of a surface of a substrate by covalent bonding of a physiologically active compound solved in a solvent-containing solution and containing one or more functional groups to the surface,
**characterized** in that the one or more functional groups of said compound are all or partly activated by a reagent in a solvent-containing first solution, whereafter the activated compound is exposed to a functional substrate surface in a solvent containing second solution during which the compound becomes covalently bonded to said surface, said reagent being a carbonylating reagent having a structural formula R₁-(CO)-R₂, wherein R₁ and R₂ represent groups which can easily be separated.

2. A method as claimed in claim 1,
**characterized** in that the carbonylating reagent is 1,1'-carbonyl diimidazole.

3. A method as claimed in claim 1,
**characterized** in that the carbonylating reagent is 1,1'-carbonyl-1,2,4-triazole.

4. A method as claimed in claims 1-3,
**characterized** in that the functional groups of the compound are carboxyl- and/or hydroxyl- and/or amino groups.

5. A method as claimed in claim 1,
**characterized** in that the functional substrate surface comprises hydroxyl- and/or carboxyl- and/or amino groups.

6. A method as claimed in claims 1-5,
**characterized** in that the compound after immobilization on the substrate surface, increases the biocompatibility of the surface.

7. A method as claimed in claim 6,
**characterized** in that the compound is heparin or a heparin-analogue.

8. A method as claimed in claim 1,
**characterized** in that the solvent of the solution for activating the compound is of an organic nature.

9. A method as claimed in claim 8,
**characterized** in that the solvent is dioxane, tetrahydrofuran, dimethylformamide, acetone, formamide or dichloroethane.

10. A method as claimed in claims 8-9,
**characterized** in that the solvent is a mixture of a plurality of solvents.

11. A method as claimed in claim 1,
**characterized** in that the substrate surface is cellulose or a cellolose derivate.

12. A method as claimed in claim 1 and claim 8,
**characterized** in that the substrate surface comprises a hydrogel.

13. A method as claimed in claim 1,
**characterized** in that the activated compound is bonded to the substrate surface via a spacer molecule.

14. A method as claimed in claim 13,
**characterized** in that a poly(ethylene oxide) containing molecule having two or more functional groups is used as the spacer molecule.

15. A method as claimed in claim 14,
**characterized** in that the functional groups are hydroxyl- and/or carboxyl- and/or amino groups.

16. A membrane comprising a surface with modified properties, a physiologically compound having been immobilized on the surface via a covalent bond,
**characterized** in that the compound has been immobilized according to the method as claimed in claims 1-15.

17. A membrane as claimed in claim 16,
**characterized** in that the membrane is made of cellulose.

18. A membrane as claimed in claim 16,
**characterized** in that the membrane is made of a cellulose derivative.

19. A membrane as claimed in claim 16,
**characterized** in that the immobilized compound increases the blood compatibility of the membrane.

20. A membrane as claimed in claim 19,
**characterized** in that the immobilized compound is a compound comprising a polyelectrolyte.

21. A membrane as claimed in claim 20,
**characterized** in that the immobilized compound is heparin.

22. A membrane as claimed in claim 20,
**characterized** in that the immobilized compound is fractionated heparin.

23. A membrane as claimed in claim 20,
**characterized** in that the immobilized compound is a chemically modified heparin.

24. A membrane as claimed in claim 21,
**characterized** in that the heparin is a heparin having a low molecular weight.

25. A membrane as claimed in claim 19,
**characterized** in that the immobilized compound is a heparin-analogue.

## Patentansprüche

1. Verfahren zur Modifizierung der Eigenschaften einer Oberfläche eines Substrats durch kovalentes Binden einer physiologisch aktiven Verbindung, die in einer Lösungsmittel enthaltenden Lösung gelöst ist und eine oder mehrere funktionelle Gruppen enthält, an die Oberfläche, dadurch gekennzeichnet, daß die eine funktionelle Gruppe oder die mehreren funktionellen Gruppen dieser Verbindung alle oder teilweise durch ein Reagens in einer Lösungsmittel enthaltenden ersten Lösung aktiviert wird/werden, woraufhin die aktivierte Verbindung einer funktionellen Substratoberfläche in einer Lösungsmittel enthaltenden zweiten Lösung ausgesetzt wird, wobei die Verbindung während dieser Zeit kovalent an die Oberfläche gebunden wird und das Reagens ein carbonylierendes Mittel mit einer Strukturformel R₁-(CO)-R₂ ist, in der R₁ und R₂ Gruppen sind, die leicht abgetrennt werden können.

2. Verfahren nach Anspruch 1, dadurch gekennzeichnet, daß das carbonylierende Reagens 1,1'-Carbonyldiimidazol ist.

3. Verfahren nach Anspruch 1, dadurch gekennzeichnet, daß das carbonylierende Reagens 1,1-Carbonyl-1,2,4-triazol ist.

4. Verfahren nach den Ansprüchen 1 bis 3, dadurch gekennzeichnet, daß die funktionellen Gruppen der Verbindung Carboxyl- und/oder Hydroxyl- und/oder Aminogruppen sind.

5. Verfahren nach Anspruch 1, dadurch gekennzeichnet, daß die funktionelle Substratoberfläche Hydroxyl- und/oder Carboxyl- und/oder Aminogruppen umfaßt.

6. Verfahren nach den Ansprüchen 1 bis 5, dadurch gekennzeichnet, daß die Verbindung nach der Immobilisierung auf der Substratoberfläche die Biokompatibilität der Oberfläche erhöht.

7. Verfahren nach Anspruch 6, dadurch gekennzeichnet, daß die Verbindung Heparin oder ein Heparin-Analogon ist.

8. Verfahren nach Anspruch 1, dadurch gekennzeichnet, daß das Lösungsmittel der Lösung für die Aktivierung der Verbindung von organischer Natur ist.

9. Verfahren nach Anspruch 8, dadurch gekennzeichnet, daß das Lösungsmittel Dioxan, Tetrahydrofuran, Dimethylformamid, Aceton, Formamid oder Dichlorethan ist.

10. Verfahren nach den Ansprüchen 8 bis 9, dadurch gekennzeichnet, daß das Lösungsmittel eine Mischung aus einer Vielzahl von Lösungsmitteln ist.

11. Verfahren nach Anspruch 1, dadurch gekennzeichnet, daß die Substratoberfläche Cellulose oder ein Cellulosederivat ist.

12. Verfahren nach Anspruch 1 und Anspruch 8, dadurch gekennzeichnet, daß die Substratoberfläche ein Hydrogel umfaßt.

13. Verfahren nach Anspruch 1, dadurch gekennzeichnet, daß die aktivierte Verbindung an die Substratoberfläche mittels eines Abstandhaltermoleküls gebunden wird.

14. Verfahren nach Anspruch 13, dadurch gekennzeichnet, daß ein Poly(ethylenoxid) enthaltendes Molekül mit zwei oder mehreren funktionellen Gruppen als Abstandhaltermolekül verwendet wird.

15. Verfahren nach Anspruch 14, dadurch gekennzeichnet, daß die funktionellen Gruppen Hydroxyl- und/oder Carboxyl- und/oder Aminogruppen sind.

16. Membran, die eine Oberfläche mit modifizierten Eigenschaften umfaßt, wobei eine physiologische Verbindung an der Oberfläche mittels einer kovalenten Bindung immobilisiert worden ist, dadurch gekennzeichnet, daß die Verbindung nach dem Verfahren gemäß den Ansprüchen 1 bis 15 immobilisiert worden ist.

17. Membran nach Anspruch 16, dadurch gekennzeichnet, daß die Membran aus Cellulose hergestellt worden ist.

18. Membran nach Anspruch 16, dadurch gekennzeichnet, daß die Membran aus einem Cellulosederivat hergestellt worden ist.

19. Membran nach Anspruch 16, dadurch gekennzeichnet, daß die immobilisierte Verbindung die Blutverträglichkeit der Membran erhöht.

20. Membran nach Anspruch 19, dadurch gekennzeichnet, daß die immobilisierte Verbindung eine Verbindung ist, die einen Polyelektrolyten umfaßt.

21. Membran nach Anspruch 20, dadurch gekennzeichnet, daß die immobilisierte Verbindung Heparin ist.

22. Membran nach Anspruch 20, dadurch gekennzeichnet, daß die immobilisierte Verbindung fraktioniertes Heparin ist.

23. Membran nach Anspruch 20, dadurch gekennzeichnet, daß die immobilisierte Verbindung ein chemisch modifiziertes Heparin ist.

24. Membran nach Anspruch 21, dadurch gekennzeichnet, daß das Heparin ein Heparin mit einem niedrigen Molekulargewicht ist.

25. Membran nach Anspruch 19, dadurch gekennzeichnet, daß die immobilisierte Verbindung ein Heparin-Analogen ist.

## Revendications

1. Procédé pour modifier les propriétés d'une surface d'un substrat par liaison covalente à la surface d'un composé physiologiquement actif dissous dans une solution contenant un solvant et contenant un ou plusieurs groupes fonctionnels, caractérisé en ce que le ou les groupes fonctionnels dudit composé sont activés totalement ou partiellement par un réactif dans une première solution contenant un solvant, après quoi le composé activé est exposé à une surface de substrat fonctionnelle dans une seconde solution contenant un solvant, période au cours de laquelle le composé devient lié de manière covalente à ladite surface, ledit réactif étant un réactif de carbonylation ayant une formule structurale R₁-(CO)-R₂ dans laquelle R₁ et R₂ représentent des groupes qui peuvent aisément être séparés.

2. Procédé selon la revendication 1, caractérisé en ce que le réactif de carbonylation est le 1,1'-carbonyldiimidazole.

3. Procédé selon la revendication 1, caractérisé en ce que le réactif de carbonylation est le 1,1'-carbonyl-1,2,4-triazole.

4. Procédé selon les revendications 1 à 3, caractérisé en ce que les groupes fonctionnels du composé sont des groupes carboxyle et/ou hydroxyle et/ou amino.

5. Procédé selon la revendication 1, caractérisé en ce que la surface de substrat fonctionnelle comprend des groupes hydroxyle et/ou carboxyle et/ou amino.

6. Procédé selon les revendications 1 à 5, caractérisé en ce que le composé, après immobilisation sur la surface du substrat, augmente la biocompatibilité de la surface.

7. Procédé selon la revendication 6, caractérisé en ce que le composé est l'héparine ou un analogue de l'héparine.

8. Procédé selon la revendication 1, caractérisé en ce que le solvant de la solution pour activer le composé est de nature organique.

9. Procédé selon la revendication 8, caractérisé en ce que le solvant est le dioxane, le tétrahydrofurane, le diméthylformamide, l'acétone, le formamide ou le dichloroéthane.

10. Procédé selon les revendications 8-9, caractérisé en ce que le solvant est un mélange de plusieurs solvants.

11. Procédé selon la revendication 1, caractérisé en ce que la surface du substrat est de la cellulose ou un dérivé cellulosique.

12. Procédé selon la revendication 1 et la revendication 8, caractérisé en ce que la surface du substrat comprend un hydrogel.

13. Procédé selon la revendication 1, caractérisé en ce que le composé activé est lié à la surface du substrat par l'intermédiaire d'une molécule espaceur.

14. Procédé selon la revendication 13, caractérisé en ce qu'une molécule contenant du poly(oxyde d'éthylène) ayant deux ou plusieurs groupes fonctionnels est utilisée comme molécule espaceur.

15. Procédé selon la revendication 14, caractérisé en ce que les groupes fonctionnels sont des groupes hydroxyle et/ou carboxyle et/ou amino.

16. Membrane comprenant une surface ayant des propriétés modifiées, un composé physiologiquement actif ayant été immobilisé sur la surface par le biais d'une liaison covalente, caractérisée en ce que le composé a été immobilisé par le procédé selon les revendications 1-15.

17. Membrane selon la revendication 16, caractérisée en ce qu'elle est constituée par de la cellulose.

18. Membrane selon la revendication 16, caractérisée en ce qu'elle est constituée par un dérivé cellulosique.

19. Membrane selon la revendication 16, caractérisée en ce que le composé immobilisé augmente la compatibilité de la membrane avec le sang.

20. Membrane selon la revendication 19, caractérisée en ce que le composé immobilisé est un composé comprenant un polyélectrolyte.

21. Membrane selon la revendication 20, caractérisée en ce que le composé immobilisé est de l'héparine.

22. Membrane selon la revendication 20, caractérisée en ce que le composé immobilisé est de l'héparine fractionnée.

23. Membrane selon la revendication 20, caractérisée en ce que le composé immobilisé est une héparine modifiée chimiquement.

24. Membrane selon la revendication 21, caractérisée en ce que l'héparine est une héparine ayant une faible masse moléculaire.

25. Membrane selon la revendication 19, caractérisée en ce que le composé immobilisé est un analogue de l'héparine.
